# EUROPÄISCHE PATENTANMELDUNG

(11) **EP 0 692 255 A2**
(43) Veröffentlichungstag der Anmeldung: **17.01.1996**
(21) Anmeldenummer: 95110224.3
(22) Anmeldetag: 30.06.1995
(51) Int. Cl.: A61K 33/42

(54) **Antacidazubereitung mit verbessertem Sicherheitsprofil**

(30) Priorität: 13.07.1994 DE 4424675
(71) Anmelder: BAYER AG, D-51368 Leverkusen (DE)
(72) Erfinder: Häusler, Franz, Dr., D-51491 Overath (DE); Scheef, Carl-Alexander, D-72076 Tübingen (DE)

(57) **Zusammenfassung**

Die Erfindung betrifft aluminiumhaltige Antacida mit Phosphatzusatz zur Reduzierung der Aluminiumresorption und Verfahren zu ihrer Herstellung.

## Beschreibung

Die Erfindung betrifft aluminiumhaltige Antacida mit Phosphatzusatz zur Reduzierung der Aluminiumresorption und Verfahren zu ihrer Herstellung.

Aluminiumhaltige Arzneizubereitungen zur peroralen Applikation, die überwiegend als Antacida eingesetzt werden, sind sehr verbreitet. Da sie häufig als nichtverschreibungspflichtige Arzneimittel, d.h. ohne ärztliche Kontrolle, eingesetzt werden, ist eine größtmögliche pharmkologische Sicherheit besonders wichtig.

Antacida werden in den unterschiedlichsten peroralen Arzneiformen wie Schlucktabletten, Kautabletten, Lutschtabletten, Brausetabletten, Kaugummis, Suspensionen oder als Gel- Arzneiformen angeboten. Sie enthalten als Wirkstoffe häufig Aluminiumverbindungen unterschiedlicher Zusammensetzungen wie z.B. basische Aluminiumoxide und -Hydroxide, bevorzugt Mischverbindungen mit Magnesium, wie z.B. Mischungen aus Aluminiumhydroxid und Magnesiumhydroxid-Gelen und Trockengelen, Schichtgitterantacida, Magnesium-Aluminium-Silikate oder Aluminium-Glycosidsulfat-Komplexe die eine ausgezeichnete antacide Wirkung sowie durch ihre spezifische Zusammensetzung geringe Nebenwirkungen besitzen. Der Vorteil aluminiumhaltiger Antacida liegt in der Fähigkeit einen therapeutisch idealen und konstanten pH-Wert im Magen einzustellen.

Die Resorption von Aluminium aus diesen Arzneimitteln wird üblicherweise als unproblematisch betrachtet, jedoch existieren Fälle, wie z.B. bei niereninsuffizenten Patienten, bei denen toxische Kumulationen von Aluminium sowie daraus resultierende Erkrankungen (z.B. Dialyse-Enzephalopathien) diagnostiziert wurden.

Aluminiumspiegel werden vor dem Hintergrund von Erkrankungen des zentralen Nervensystems zunehmend kritischer beobachtet, so daß eine möglichst geringe Belastung des menschlichen Organismus mit diesem Leichtmetall erwünscht ist, auch wenn Überschreitungen des Normwertes von 1-35 µg/l Serum bei Gesunden unter Antacidatherapie bislang nicht diagnostiziert wurden. In den deutschen Aufbereitungsmonographien für den humanmedizinischen Bereich, veröffentlicht 1993 wird ein maximaler Aluminiumspiegel von 40 µg/l Serum festgelegt. Bei Überschreiten dieses Grenzwertes sollte die Therapie abgebrochen werden.

Aluminiumverbindungen als amphotere Verbindungen lösen sich im Sauren deutlich besser als im neutralen Milieu. Daher wird im Magen immer ein Teil des Aluminiums unter Neutralisation der Magensäure gelöst. Diese Fähigkeit wird bei den aluminiumhaltigen Antacida therapeutisch genutzt. Bei Antacida die lediglich Aluminiumverbindungen enthalten korreliert die Löslichkeit im sauren Milieu mit der antaciden Wirksamkeit.

Aluminiumverbindungen werden auch als Phosphatbinder bei Patienten mit Niereninsuffizienz, d.h. verminderter renaler Clearance eingesetzt, um die Resorption von Phosphat aus dem Gastro-Intestinal-Trakt und so eine Hyperphosphatämie zu verhindern. Unter Dauertherapie mit Antacida wurden jedoch bei Patienten mit normaler renaler Clearance verminderte Phosphatspiegel im Serum diagnostiziert, die zu Osteomalazie, Hypophosphatämie, Hypophosphaturie sowie anderen Phosphatmangelerkrankungen führen. Um eine Phosphatverarmung unter Dauertherapie zu vermeiden, beschreibt EP 0 040 364 synthetisierte Hydrotalcite, in denen Carbonatgruppen durch Phosphatgruppen ersetzt wurden und somit die Resorption von Phosphat aus dem Gastro-Intestinal-Trakt weniger reduziert wird.

Um die Aluminiumresorption aus aluminiumhaltigen Antacida gering zu halten existieren prinzipiell zwei Möglichkeiten. Eine Möglichkeit ist ein Antacidum mit einem möglichst geringen Anteil an Aluminium einzusetzen. Eine zweite Möglichkeit ist, das Aluminium in möglichst schwer lösliche Form zuzuführen, um so eine Resorption zu verhindern.

Vielfach werden Aluminium-Magnesium-Mischverbindungen eingesetzt, die sich durch einen geringeren Aluminiumanteil bei ausgezeichneter antacider Wirksamkeit auszeichnen. Diese Verbindungen reagieren schnell und besitzen ein hohes Säureneutralisationsvermögen, neben anderen substanzspezifischen Vorteilen dieser Stoffklasse. Beispiele dieser Substanzklasse sind Hydrotalcit, Magaldrat sowie die Gel-Antacida aus Gemischen von Magnesium-Aluminium-Hydroxiden sowie deren Trockengele. Ihr Aluminiumgehalt reicht von ca. 5,1 mg/mEq bei Aluminium-Magnesium-Hydroxid Gemischen bis zu 3 mg/mEq bei Hydrotalcit. Abhängig von der zugeführten Aluminiummenge kann jedoch bei Therapie Gesunder die obere physiologische Grenze erreicht werden, oder insbesondere bei Vorliegen von Störungen der renalen Sekretion diesen Grenzwert von 40 µg/l Serum überschreiten, wobei insbesondere zu beachten ist, daß bei älteren Menschen die Nierenfunktion oftmals eingeschränkt ist.

Zu den schwerlöslichen Aluminiumverbindungen zählen die Aluminiumphosphate. Diese werden auch direkt therapeutisch eingesetzt und zeigen nach Applikation geringe Aluminiumserumspiegel. Aluminiumphosphate zeigen in der Therapie jedoch eine sehr schwache Säureneutralisationskapazität und schlechte Neutralisationsgeschwindigkeit, so daß diese Zubereitungen oftmals nicht den pH-Wert des Magens in den therapeutisch optimalen Bereich zwischen pH 3 und 5 heben können. Dafür müßten überproportional große Mengen eingesetzt werden, womit die zugeführte Menge Aluminium ebenfalls erhöht wird Damit sind diese Verbindungen für eine moderne Antacida-Therapie nicht mehr zeitgemäß.

Es wurde gefunden, daß nicht nur bei Einsatz von schwerlöslichen Aluminiumphosphaten, sondern auch bei Zusatz von Phosphaten in Form anderer Salze zu aluminiumhaltigen Antacida der Anteil der zur Resorption zu Verfügung stehenden Aluminiumionen signifikant verringert werden konnte, ohne jedoch die Neutralisationskapazität des aluminiumhaltigen Antacidums zu vermindern. Dabei wurde unerwartet festgestellt, daß die Verminderung der resorbierbaren Aluminiumionen von den Kationen des Phosphatsalzes abhängig ist, wobei sich insbesondere die Alkaliphosphate für nichtbrausende Zubereitungen als geeignet erwiesen haben. Bei brausenden Zubereitungen wirken dagegen die Erdalkaliphosphate überraschenderweise deutlich effektiver als die Alkaliphosphate. Verschiedene Phosphatsalze haben dabei unerwarteterweise einen signifkanten Einfluß auf das Ausmaß der Reduktion freier Aluminiumionen nach einer simulierten Magen-Darm-Passage.

Die Bestimmung der freien Aluminiumionen wurde folgendermaßen durchgeführt:
Die Formulierungsbestandteile werden in einer definierten Menge Wasser suspendiert und bis zu einem konstanten pH gerührt. Zur Simulation einer Magen-Darm-Passage wird mit kunstlichem Magensaft bis pH 1 titriert und dann mit künstlichem Darmsaft bis pH 8 zurücktitriert. Die Suspension wird zentrifugiert und in 20 ml des klaren Überstandes der Gehalt an gelöstem Aluminium bestimmt. Der prozentuale Anteil freies Aluminium bezieht sich auf die eingesetzte Menge Aluminium des Arzneistoffes und stellt der Aluminiumaustrag aus dem Arzneistoff während einer simulierten Magen-Darm-Passage dar.

| | **Inhaltsstoffe** | **Masse (mg)** | **freies Aluminium (%)** |
|---|---|---|---|
| 1. | Hydrotalcit | 500 | 0,22 |
| 2. | Hydrotalcit | 500 | 0,23 |
| | Tricalciumphosphat | 257 | |
| 3. | Hydrotalcit | 500 | 0,04 |
| | Kaliumphosphat | 352 | |
| 4. | Hydrotalcit | 500 | 0,13 |
| | Trimagnesiumphosphat | 224 | |
| 5. | Hydrotalcit | 500 | 93,9 |
| | Citronensäure | 240 | |
| | Natriumbicarbonat | 1.344 | |
| 6. | Hydrotalcit | 500 | 63,20 |
| | Citronensäure | 240 | |
| | Natriumbicarbonat | 1.344 | |
| | Kaliumdihydrogenphosphat | 1.088 | |
| 7. | Hydrotalcit | 500 | 9,2 |
| | Citronensäure | 384 | |
| | Natriumbicarbonat | 504 | |
| | Calciumphosphat | 930 | |

Das entsprechende Phosphatsalz zur Senkung des Aluminiumaustrags wird nach üblichen Methoden und ohne aufwendige Verarbeitungsschritte den entsprechenden Antacida-Rezepturen hinzugefügt. Die Menge des zugefügten Phosphats richtet sich dabei nach der Menge im Gastro-Intestinal-Trakt freigesetzten Aluminiums, also dem Aluminiumgehalt der Aluminiumverbindung und sollte der im Gastro-Intestinal-Trakt freigesetzten Menge Aluminiumionen in etwa äquivalent sein. Vorzugsweise wird eine Phosphatmenge gewählt, die der 0,5- bis 1,5fachen der in der Formulierung verarbeiteten Aluminiummenge äquivalent ist.

### Ausführungsbeispiele

### Beispiel 1

| | |
|---|---|
| Hydrotalcit | 500 mg |
| Kaliumphosphat | 250 mg |
| Maisstärke | 240 mg |
| kolloidale Kieselsäure | 5 mg |
| Magnesiumstearat | 5 mg |

Hydrotalcit, Kaliumphosphat und Maisstärke werden in einem Rotorgranulator mit Wasser granuliert. Das Granulat wird in einem Trockenschrank getrocknet, klassiert und mit der kolloidalen Kieselsäure und dem Magnesiumstearat gemischt und zu Tabletten von 1 g Gewicht verpreßt.

### Beispiel 2

| | |
|---|---|
| Aluminiumhydroxid | 250 mg |
| Magnesiumhydroxid | 250 mg |
| Trimagnesiumphosphat | 250 mg |
| Avicel | 195 mg |
| Aerosil | 5 mg |
| Talkum | 50 mg |

Die Bestandteile werden alle in einen geeigneten Mischer eingewogen und gründlich gemischt und dann direkt zu Tabletten von 1 g Gewicht verpreßt.

### Beispiel 3

| | |
|---|---|
| Almagat | 500 mg |
| Kaliumphosphat | 350 mg |
| Lactose | 270 mg |

Almagat, Kaliumphosphat und Lactose werden in ein Tellergranulator eingewogen und mit einer gesättigten Lactoselösung granuliert. Das Granulat wird getrocknet und klassiert und dann als Einzeldosen in Polypropylenbeutel à 1 g verpackt.

### Beispiel 4

| | |
|---|---|
| Hydrotalcit | 500 mg |
| Trimagnesiumphosphat | 500 mg |
| Citronensäure | 1.120 mg |
| Natriumbicarbonat | 1.344 mg |
| Polyethylenglykol 6000 | 46 mg |

Citronensäure und Natriumbicarbonat werden mit einer Ethanol-Wasser-Mischung granuliert, das Hydrotalcit und das Trimagnesiumphosphat werden zugemischt und als letztes wird das Polyethylenglykol 6000 untergemischt und die Mischung unter Klimatisierten Bedingungen zu Tabletten à 3.500 mg verpreßt.

## Patentansprüche

1. Aluminiumhaltige Arzneizubereitung zu peroralen Applikation mit verringerter Resorption von Aluminiumionen, dadurch gekennzeichnet, daß sie 0,5 bis 1,5 Moläquivalent Phosphat enthält.

2. Arzneizubereitung gemäß Anspruch 1 in Form von Schlucktabletten, Kautabletten, Lutschtabletten, Brausetabletten, Kaugummis, Suspensionen und Gel-Arzneiform zur oralen Anwendung.

3. Arzneizubereitung gemäß Anspruch 1, dadurch gekennzeichnet, daß man Alkaliphosphate in nichtbrausenden Arzneiformen und Erdalkaliphosphate in brausenden Arzneiformen einsetzt.

4. Arzneiformen gemäß Anspruch 1, dadurch gekennzeichnet, daß sie aluminiumhaltige Antacida aus der Gruppe Aluminiumhydroxide, Aluminiumhydroxid-Magnesium-Mischverbindungen, Aluminium-Magnesium-Silikate, aluminiumhaltige Verbindungen mit kristalliner Struktur oder basische Aluminium-Glykosidsulfat-Komplexe als Wirkstoff enthalten.

5. Verwendung von Alkali- und Erdalkaliphosphaten bei der Herstellung von aluminiumhaltigen Arzneizubereitungen zur oralen Applikation, zur Verminderung der Aluminiumresorption.

6. Verfahren zur Herstellung von Arzneizubereitungen gemäß Anspruch 1, dadurch gekennzeichnet, daß man mindestens einen aluminiumhaltigen Wirkstoff, gegebenenfalls unter Verwendung von üblichen Hilfs- und Trägerstoffen mit einer der verwendeten Aluminiummenge in etwa äquivalenten Menge von Alkali- oder Erdalkaliphosphaten vermischt.
